# EUROPEAN PATENT SPECIFICATION

(11) **EP 3 152 218 B1**
(45) Date of publication and mention of the grant of the patent: **26.12.2018**
(21) Application number: 15725660.3
(22) Date of filing: 02.06.2015
(51) Int. Cl.: C07F 17/00, B01J 31/22, C08F 4/659, C08F 4/6592, C07C 49/115, C07C 13/465, C07C 21/06

(54) **PROCESS FOR SYNTHESIS OF INDENES**
VERFAHREN ZUR SYNTHESE VON INDENEN
PROCÉDÉ DE SYNTHÈSE D'INDÈNES

(30) Priority: 09.06.2014 GB 201410202
(43) Date of publication of application: 12.04.2017
(73) Proprietor: SCG Chemicals Co., Ltd., Bangkok 10800 (TH)
(72) Inventor: O'HARE, Dermot, Oxford Oxfordshire OX1 3TA (GB); BUFFET, Jean-Charles, Oxford Oxfordshire OX1 3TA (GB); ARNOLD, Thomas, Oxford Oxfordshire OX1 3TA (GB); MAYALARP, Vichitt, Bangkok 10800 (TH)
(74) Representative: HGF Limited
(86) International application number: PCT/EP2015/062284
(87) International publication number: WO 2015/189073

(56) References cited:
- WO-A1-2011/051705
- READY T E ET AL: "New indenyl titanium catalysts for syndiospecific styrene polymerizations", JOURNAL OF ORGANOMETALLIC CHEMISTRY, ELSEVIER-SEQUOIA S.A. LAUSANNE, CH, vol. 583, no. 1-2, 30 June 1999 (1999-06-30), pages 11-27, XP004172733, ISSN: 0022-328X, DOI: 10.1016/S0022-328X(99)00095-9

## Description

### INTRODUCTION

The present invention relates to a process for the synthesis of 2,3,4,5,6,7-substituted indenes, which are useful precursors for the formation of certain *ansa-*metallocene catalysts.

### BACKGROUND OF THE INVENTION

It is well known that ethylene (and α-olefins in general) can be readily polymerized at low or medium pressures in the presence of certain transition metal catalysts. These catalysts are generally known as Ziegler-Natta type catalysts.

A particular group of these Ziegler-Natta type catalysts, which catalyse the polymerization of ethylene (and α-olefins in general), comprise an aluminoxane activator and a metallocene transition metal catalyst. Metallocenes comprise a metal bound between two η⁵-cyclopentadienyl type ligands. Generally the η⁵-cyclopentadienyl type ligands are selected from η⁵-cyclopentadienyl, η⁵-indenyl and η⁵-fluorenyl.

It is also well known that these η⁵-cyclopentadienyl type ligands can be modified in a myriad of ways. One particular modification involves the introduction of a linking group between the two cyclopentadienyl rings to form ansa-metallocenes.

WO2011/051705 describes certain *ansa*-metallocene catalysts that demonstrate particularly high catalytic performance when utilised for the polymerization of polyethylene. The catalysts described in WO2011/051705 comprise a metal atom bound between two inter-linked indenyl moieties. The indenyl moieties bear substituents in the 2,3,4,5,6 and 7-positions of each indenyl moiety, and a linking group (such as -CH₂-CH₂-) connects the 1-positions of the respective indenyl moieties together.

In the synthesis of such *ansa*-metallocene catalysts, the desired 2,3,4,5,6,7-substituted indene precursor is initially formed and then the two indenyl moieties are cross-linked by the insertion of the linking group that connects the 1-positions of two indenyl moieties together. The resultant linked bis(2,3,4,5,6,7-substituted indenyl) ligand is then complexed with the desired metal to form the *ansa*-metallocene catalyst.

As part of this synthetic procedure, there is a need for a simple, efficient and scalable process for the formation of the desired 2,3,4,5,6,7-substituted indene precursors.

O'Hare et al. (Organometallics 1992, 11, 48) describes a process for the formation of a heptamethylindene as a precursor for the formation of certain metallocene catalysts [namely bis(heptamethylindenyl)iron(II), bis(heptamethylindenyl)-iron(III) hexafluorophosphate, bis(heptamethylindenyl)cobalt(III) hexafluorophosphate and bis(heptamethylindenyl)cobalt(II)].

The process described by O'Hare *et al.* involves a number of different reaction steps. The first step involves reacting tiglic acid with thionyl chloride under reflux for 5 hours. Excess thionyl chloride was evaporated off at 78 °C. The product was then distilled under reduced pressure (10 mmHg, ca. 37 °C) to give a colourless liquid (tigloyl chloride) in a 95% yield. In a subsequent step, a mixture of AlCl₃ is stirred in CS₂ at -5 °C and a mixture of 1,2,3,4-tetramethylbenzene and tigloyl chloride were added slowly over a period of 1 hour. After the addition, the mixture turned red-brown and solid CS₂ was added. The mixture was warmed to room temperature and stirred overnight. It was then heated to 50 °C for 2 hours and then poured into a mixture of ice and concentrated HCl before extracting with diethyl ether and drying the ether layer over CaCl₂. Upon distillation, a dark brown crude liquid was obtained, which was further distilled under reduced pressure giving a yellow oily liquid (2,3,4,5,6,7-hexamethlyindan-1-one ketone) in 85% yield. In a further step, the ketone is reacted with LiMe to form heptamethylindene.
A process for the preparation of 1,2,4,5,6,7-hexamethylindene is disclosed by Thomas E. Ready et al. in Journal of Organometallic Chemistry 583 (1999) 11-27.

While the process described above enables the desired indene products to be formed, there remains a need for improved methods of forming the particular 2,3,4,5,6,7-substituted indenes that can be used for the formation of *ansa-*metallocene catalysts, such as those described in WO2011/051705. In particular, there is a need for improved synthetic methods that are simple, efficient, provide good product purity and which are suitable for scale-up.

### SUMMARY OF THE INVENTION

According to a first aspect of the present invention there is provided a process for the preparation of a compound of the formula I shown below: 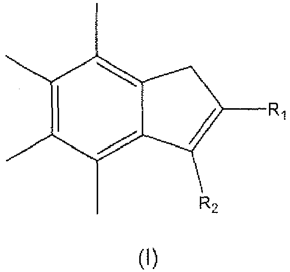
wherein R₁ and R₂ are each (1-10C)alkyl;
and wherein the process comprises the steps of:
   (i) reacting a compound of formula A: 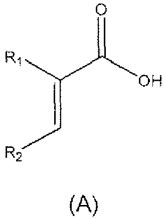
      wherein R₁ and R₂ are each as defined above;
      with a chlorinating or brominating agent to form a compound of formula B: 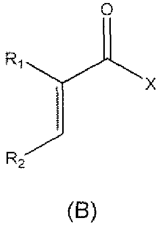 wherein R₁ and R₂ are each as defined above and X is chloro or bromo;
      and then adding 1,2,3,4-tetramethyibenzene and a Lewis acid catalyst to the reaction mixture to react with compound B to form a compound of formula C: 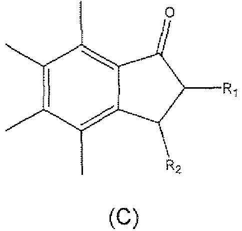 wherein R₁ and R₂ are each as defined above;
   (ii) reacting the compound of formula C with a solution of a hydride transfer reagent to reduce the ketone to the corresponding alcohol and then by adding a dehydrating agent to the reaction mixture to form a compound of formula I, namely dehydrating the alcohol.

Step (i) of the process defined above is a "one-pot" step. Thus, when it is stated in step (i) that 1,2,3,4-tetramethylbenzene and a Lewis acid catalyst are added to the reaction mixture to react with compound B to form a compound of formula C, it is meant that the 1,2,3,4-tetramethylbenzene and a Lewis acid catalyst are added directly to the reaction mixture comprising the compound of formula B. It is not necessary to isolate the compound of formula B prior to the addition of 1,2,3,4-tetramethylbenzene and the Lewis acid catalyst.

Suitably, step (ii) is also a "one-pot" reaction in which the dehydration of the alcohol formed by the reduction of the ketone of formula C is facilitated by the addition of a dehydrating agent directly to the reaction mixture comprising the alcohol (the reduced ketone). It is not necessary to isolate the alcohol (the reduced ketone) prior to the addition of the dehydrating agent.

According to a second aspect of the present invention there is provided a process for the preparation of a compound of the formula C as defined above,
wherein the process comprise the steps of:
(i) reacting a compound of formula A: 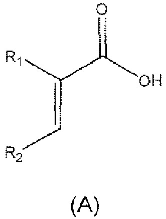
   wherein R₁ and R₂ are each as defined above;
   with a chlorinating or brominating agent to form a compound of formula B: 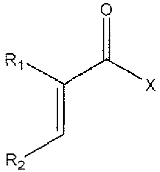 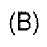 wherein R₁ and R₂ are each as defined above and X is chloro or bromo;
   and then adding 1,2,3,4-tetramethylbenzene and a Lewis acid catalyst to the reaction mixture to react with compound B to form a compound of formula C: 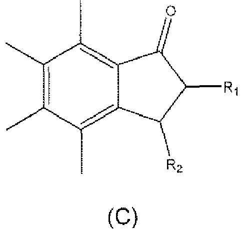 wherein R₁ and R₂ are each as defined above.

According to a third aspect of the present invention there is provided a process of forming a compound of Formula I as defined herein, said process comprising reacting a compound of formula C as defined herein with a solution of a hydride transfer reagent to reduce the ketone to the corresponding alcohol and then dehydrating the alcohol to form a compound of Formula I as defined herein.

According to a fourth aspect of the present invention there is provided a process for forming a compound of formula II 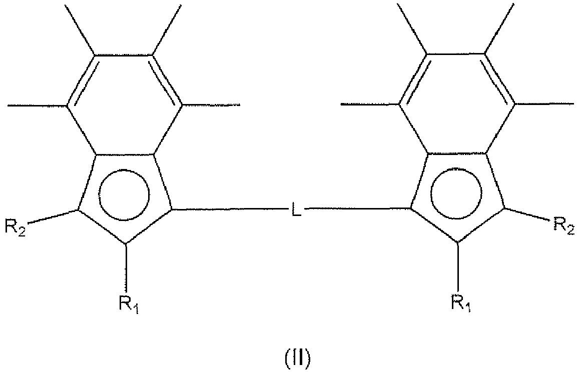 wherein
R₁, R₂ are as defined herein; and
L is a bridging group of the formula -[C(R^{x}R^{y})]ₙ- wherein n is 1, 2 or 3 and R^{x} and R^{y} are each independently hydrogen, (1-6C)alkyl(2-6C)alkenyl, (2-6C)alkynyl or (1-6C)alkoxy; or a group -SiRₐR_{b} wherein Rₐ and R_{b} are each independently selected from (1-6C)alkyl, (2-6C)alkenyl, (2-6C)alkynyl, (1-6C)alkoxy or phenyl;
wherein the process comprises:
(i) forming a compound of formula I by a process as defined in the first aspect of the invention;
(ii) reacting the compound of formula I with an organolithium, organosodium or organopotassium compound of the formula:

   MQ

   wherein M is lithium, sodium, or potassium and Q is an (1-6C)alkyl or aryl group;
   to form a compound of formula D shown below: 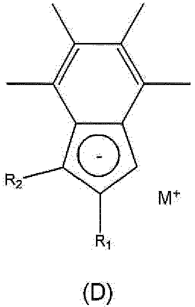
(iii) reacting two equivalents of a compound of formula D shown above with one equivalent of a compound having formula E shown below:

   Z₁-L-Z₂ (E)

   wherein L is as defined hereinbefore and Z₁ and Z₂ are suitable leaving groups (such as halo (e.g. chloro or bromo)) to form a compound of formula II.

According to a fifth aspect of the present invention there is provided a process for forming a compound of formula III 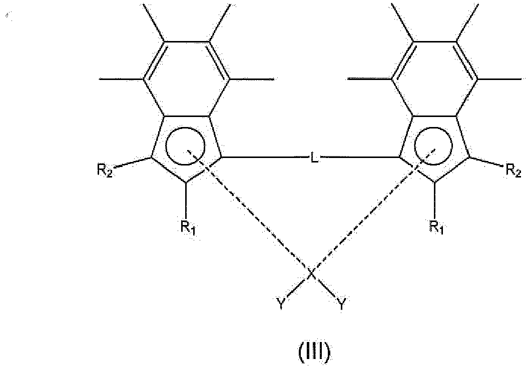
wherein R₁, R₂ and L are as defined herein;
X is zirconium, hafnium or titanium;
Y is selected from halo, hydride, a phosphonated or sulfonated anion, or a (1-6C)alkyl, (1-6C)alkoxy, aryl or aryloxy group which is optionally substituted with halo, nitro, amino, phenyl, (1-6C)alkoxy, or Si[(1-4C)alkyl]₃.
wherein the process comprises:
   (i) forming a compound of formula II by the process defined above in the fourth aspect of the present invention;
   (ii) reacting the compound of formula II with a compound of the formula:

      X(Y)₂(Z₃)₂

      wherein X and Y are as defined above and Z₃ is a suitable leaving group (e.g. chloro).

The present invention also relates to a compound of formula I, II or III as defined herein obtainable by, obtained by, or directly obtained by any one of the processes defined herein.

Preferred embodiments are disclosed in the sub-claims.

### DETAILED DESCRIPTION OF THE INVENTION

### Definitions

### Alkyl

The term "alkyl" as used herein includes reference to straight or branched chain alkyl moieties, typically having 1, 2, 3, 4, 5 or 6 carbon atoms. This term includes reference to groups such as methyl, ethyl, propyl (n-propyl or isopropyl), butyl (n-butyl, sec-butyl or tert-butyl), pentyl, hexyl and the like. In particular, an alkyl may have 1, 2, 3 or 4 carbon atoms.

### Alkenyl

The term "alkenyl" as used herein includes reference to straight or branched chain alkenyl moieties, typically having 1, 2, 3, 4, 5 or 6 carbon atoms. The term includes reference to alkenyl moieties containing 1, 2 or 3 carbon-carbon double bonds (C=C). This term includes reference to groups such as ethenyl (vinyl), propenyl (allyl), butenyl, pentenyl and hexenyl, as well as both the *cis* and *trans* isomers thereof.

### Alkynyl

The term "alkynyl" as used herein includes reference to straight or branched chain alkynyl moieties, typically having 1, 2, 3, 4, 5 or 6 carbon atoms. The term includes reference to alkynyl moieties containing 1, 2 or 3 carbon-carbon triple bonds (C≡C). This term includes reference to groups such as ethynyl, propynyl, butynyl, pentynyl and hexynyl.

### Alkoxy

The term "alkoxy" as used herein includes reference to -O-alkyl, wherein alkyl is straight or branched chain and comprises 1, 2, 3, 4, 5 or 6 carbon atoms. In one class of embodiments, alkoxy has 1, 2, 3 or 4 carbon atoms. This term includes reference to groups such as methoxy, ethoxy, propoxy, isopropoxy, butoxy, tert-butoxy, pentoxy, hexoxy and the like.

### Aryl

The term "aryl" as used herein includes reference to an aromatic ring system comprising 6, 7, 8, 9 or 10 ring carbon atoms. Aryl is often phenyl but may be a polycyclic ring system, having two or more rings, at least one of which is aromatic. This term includes reference to groups such as phenyl, naphthyl and the like.

### Halogen

The term "halogen" or "halo" as used herein includes reference to F, Cl, Br or I. In a particular, halogen may be F or Cl, of which Cl is more common.

### Substituted

The term "substituted" as used herein in reference to a moiety means that one or more, especially up to 5, more especially 1, 2 or 3, of the hydrogen atoms in said moiety are replaced independently of each other by the corresponding number of the described substituents. The term "optionally substituted" as used herein means substituted or unsubstituted.

It will, of course, be understood that substituents are only at positions where they are chemically possible, the person skilled in the art being able to decide (either experimentally or theoretically) without inappropriate effort whether a particular substitution is possible. For example, amino or hydroxy groups with free hydrogen may be unstable if bound to carbon atoms with unsaturated (e.g. olefinic) bonds. Additionally, it will of course be understood that the substituents described herein may themselves be substituted by any substituent, subject to the aforementioned restriction to appropriate substitutions as recognised by the skilled person.

### Compounds of formula I, II and III

As indicated above, the present invention relates to preparation of compounds of formula I, II and III by the processes defined herein.

In the compounds of formula I, II and III defined herein, R₁ and R₂ are each independently selected from (1-10C)alkyl. Suitably, R₁ and R₂ are each independently selected from (1-3C)alkyl. In an embodiment, R₁ and R₂ are each independently selected from (1-2C)alkyl. In a particular embodiment, R₁ and R₂ are both methyl.

Suitably, Rₐ and R_{b} are each independently selected from (1-4C)alkyl, (2-4C)alkenyl or (2-4C)alkynyl. Even more suitably, Rₐ and R_{b} are each independently selected from methyl, propyl and allyl. Most suitably, Rₐ and R_{b} are both methyl.

In an embodiment, L is -CH₂-CH₂-, -CH₂-CH₂-CH₂- or a group -SiRₐR_{b} wherein Rₐ and R_{b} are each independently selected from methyl, propyl and allyl.

In an embodiment, L is -CH₂-CH₂-, -CH₂-CH₂-CH₂- or a group -SiRₐR_{b} wherein Rₐ and R_{b} are each independently selected from methyl and allyl.

In an embodiment, L is -CH₂-CH₂-, -CH₂-CH₂-CH₂- or a group -SiMe₂ or-Si(Me)(allyl).

In a particular embodiment, L is -CH₂-CH₂- or a group -SiMe₂ or -Si(Me)(allyl).

In a particular embodiment, L is -CH₂-CH₂- or -SiMe₂.

In an embodiment, X is zirconium or hafnium.

In a particular embodiment, X is zirconium.

In a particular embodiment, X is hafnium.

In an embodiment, each Y group is the same.

In an embodiment, Y is selected from halo, (1-6C)alkyl or phenyl, wherein the alkyl or phenyl group is optionally substituted with halo, nitro, amino, phenyl, (1-6C)alkoxy, or Si[(1-4C)alkyl]₃,

In an embodiment, Y is selected from halo or a (1-6C)alkyl group which is optionally substituted with halo, nitro, amino, phenyl, (1-6C)alkoxy, or Si[(1-4C)alkyl]₃.

In another embodiment, Y is selected from halo or a (1-6C)alkyl group which is optionally substituted with halo, phenyl, or Si[(1-2C)alkyl]₃.

In another embodiment, Y is selected from chloro, bromo, or a (1-4C)alkyl group which is optionally substituted with halo, phenyl, or Si[Me]₃.

In a particular embodiment, Y is selected from chloro or a (1-4C)alkyl group which is optionally substituted with phenyl or Si[Me]₃.

In a further embodiment, Y is chloro, bromo or methyl.

In a further embodiment, Y is chloro or bromo.

In a further embodiment, Y is chloro.

In another embodiment, Y is methyl.

In a particular embodiment, R₁ and R₂ are both methyl; L is -CH₂-CH₂-, -CH₂-CH₂-CH₂- or a group -SiRₐR_{b} wherein Rₐ and R_{b} are each independently selected from methyl, propyl and allyl; X is zirconium or hafnium; and Y is selected from chloro, bromo, or a (1-4C)alkyl group which is optionally substituted with halo, phenyl or Si[Me]₃.

In a particular embodiment, R₁ and R₂ are both methyl; L is -CH₂-CH₂-, -CH₂-CH₂-CH₂- or a group -SiRₐR_{b} wherein Rₐ and R_{b} are each independently selected from methyl and allyl; X is zirconium or hafnium; and Y is selected from chloro, bromo, or a (1-4C)alkyl group.

### Process for producing compounds of formula I

As described hereinbefore, the present invention provides a process for the preparation of a compound of the formula I shown below: 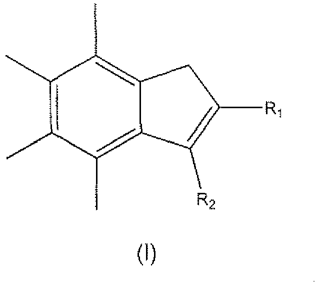 comprising the steps (i) and (ii) defined above.

The process of the present invention provides a number of distinct advantages. Firstly, steps (i) and (ii) of the process defined herein for the first aspect of the present invention are both discrete "one-pot" steps. This provides a more facile process when compared to the synthetic approaches described in the prior art. It also provides some further advantages.

For example, when compared with the process described by O'Hare et al. (Organometallics 1992, 11, 48), step (i) of the process of the present invention has the advantages of:
- The reaction times and temperatures can be reduced, thereby making the reaction more cost effective and more suitable for scale-up.
- The need for distillation is negated, which is time consuming and energy intensive
- The process can use different chlorinating agents (such as, for example, oxalyl chloride) to the SOCl₂ used in the O'Hare *et al.* method which can be hazardous because it causes severe burns.
- The use of CS₂ can also be avoided, which is flammable, toxic and has a very low flash point.
- The reaction can be carried out at room temperature, if so desired.
- The process of the present invention also enables the ketone (i.e. the compound of formula C) to be produced with high yield and with improved purity (in the example described herein, hexamethylindanone (Ind^{#}=O) is obtained with 96% yield).

In addition, the overall yield of the process is high (94% in the example provided herein) and the purity of the product is also high.

In step (ii), the use of a solution of a hydride transfer agent has been found to reduce the reaction time for the reduction of the ketone. Furthermore, the time required for the dehydration of the reduced ketone to form the compound of formula I is also reduced by the "one-pot" step (ii) reaction employed in the process of the present invention.

The first step [step (i)] of the reaction involves reacting a compound of formula A: 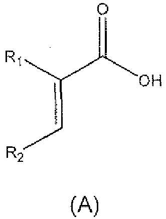
wherein R₁ and R₂ are each as defined herein;
with a chlorinating or brominating agent to form a compound of formula B: 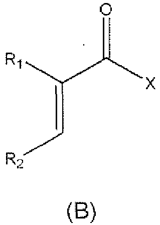 wherein R₁ and R₂ are each as defined herein and X is chloro or bromo;
and then adding 1,2,3,4-tetramethylbenzene and a Lewis acid catalyst to the reaction mixture to react with compound B to form a compound of formula C:

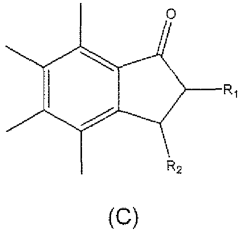

This whole step is carried out as a "one-pot" reaction with the initial reaction to form compound B followed by the addition of the required reagents (1,2,3,4-tetramethylbenzene and a Lewis acid catalyst) to subsequently form compound C.

The reactions that make up step (i) may be carried out in any suitable solvent. A person skilled in the art will know how to select suitable solvents for this reaction. Nontoxic polar solvents such as dichloromethane, THF, ethers (e.g. methyl t-butyl ether (MtBE) or dioxane) etc. are examples of solvents that can be used. In a particular embodiment, the solvent is dichloromethane.

A person skilled in the art will be able to select suitable reaction conditions (reaction atmosphere, reaction temperature, duration of the reaction and workup procedures) for this reaction.

Suitably, the process of step (i) may be carried out at room temperature (e.g. 20-25 °C) or the reaction mixture may be heated, for example, up to a temperature of up to 80 °C depending on the solvent that is used.

The duration of step (i) is suitably between 1 and 24 hours, with reaction times of 16 hours or less being generally preferred.

Suitably, step (i) is carried out under an inert atmosphere, such as, for example, a nitrogen atmosphere.

Any suitable chlorinating or brominating agent may be used for the reaction with the compound of formula A. Suitably, the chlorinating agent is selected from the group consisting of oxalyl chloride, PCl₃, PCl₅ and SOCL₂ and the brominating agent is selected from the group consisting of PBr₃ and PBr₅.

In an embodiment, a chlorinating agent is used to form compounds of formula B in which X is chloro. In a further embodiment, a chlorinating agent selected from oxalyl chloride, PCl₃ and/or PCl₅. In a particular embodiment, oxalyl chloride is used as the chlorinating agent.

The reaction between the compound of formula A and the chlorinating agent may take from 1 to 20 hours. Suitably, the reaction takes place at room temperature for up to 15 hours or, more preferably, up to 12 hours.

Following the reaction between the compound of formula A and the chlorinating or brominating agent to form a compound of formula B, a Lewis acid catalyst is added to the reaction mixture along with 1,2,3,4-tetramethylbenzene.

Any suitable Lewis acid catalyst may be used. In an embodiment, the Lewis acid catalyst is selected from AlCl₃, AlBr₃ and BCl₃. in a particular embodiment, the Lewis acid catalyst is AlCl₃.

Suitably, the Lewis acid catalyst is added to the reaction mixture prior to the 1,2,3,4-tetramethylbenzene. The reaction mixture may be cooled, for example to less than 10°C, prior to the addition of the Lewis acid catalyst.

The reaction between the compound of formula B and the 1,2,3,4-tetramethylbenzene is allowed to proceed for up to 6 hours, and more preferably up to 4 hours.

The reaction may be quenched by the addition of an acid, for example concentrated hydrochloric acid, and optionally cooling the reaction mixture, for example by the addition of ice.

The product, namely the compound of formula C is then extracted from the reaction mixture using standard techniques.

In step (ii) of this process, the compound of formula C is reacted with a solution of a hydride transfer reagent to reduce the ketone to the corresponding alcohol, which is then dehydrated to form a compound of Formula I as defined herein.

Again, this reaction can be carried out as a "one-pot" reaction. The first part of this reaction step involves the reduction of the compound of formula C. This provides a compound of the formula CR:

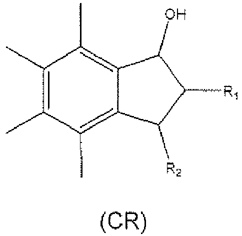

This reduction is achieved by using a solution of a hydride transfer reagent. Suitable examples of hydride transfer reagents include LiAlH₄ and NaBH₄. In a particular embodiment, the hydride transfer reagent is a solution of LiAlH₄ in a suitable solvent, such as, for example, THF.

This reaction is suitably carried out in an inert atmosphere, for example a nitrogen atmosphere. The reduction reaction suitably proceeds for up to 6 hours, and more preferably for up to 4 hours.

The reaction may proceed at any suitable temperature. For example, temperatures within the range of 0 to 30 °C may be used. Suitably, the solution of the hydride transfer reagent is cooled, for example to less than 10 °C, prior to the addition of the compound of formula C.

The reduction reaction is suitably quenched, for example by the addition of water. The reduced compound of formula C (compound CR) is then dehydrated by the addition of a dehydrating agent. Any suitable dehydrating agent may be used. Suitably, the dehydrating agent is an acid, for example concentrated sulphuric, hydrochloric or phosphoric acid. The direct addition of the dehydrating agent to the reaction mixture efficiently dehydrates the reduced form of the compound of formula C (compound CR) to provide the desired compound of formula I.

The reaction time for the dehydration step is typically up to 60 minutes, and more preferably up to 30 minutes.

The desired compound of formula I is then extracted using standard techniques.

### Process for producing compounds of formula II

According to a fourth aspect of the present invention there is provided a process for forming a compound of formula II 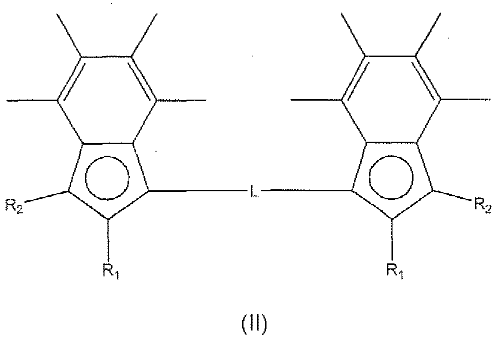 wherein
R₁, R₂ and L are as defined herein.

The process involves:
(i) forming a compound of formula I by a process as defined herein;
(ii) reacting the compound of formula I with an organolithium, organosodium or organopotassium compound of the formula:

   MQ

   wherein M is lithium, sodium, or potassium and Q is an (1-6C)alkyl or aryl group;
   to form a compound of formula D shown below: 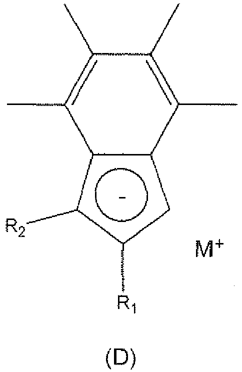
(iii) reacting two equivalents of a compound of formula D shown above with one equivalent of a compound having formula E shown below:

   Z₁-L-Z₂ (E)

   wherein L is as defined hereinbefore and Z₁ and Z₂ are suitable leaving groups (such as halo (e.g. chloro or bromo)) to form a compound of formula II.

Step (i) of this process is defined hereinbefore.

In step (ii) of this process, the compound of formula I is suitably reacted with an organolithium compound (i.e. M is lithium). An example of a suitable organolithium compound is n-butyllithium.

Suitably, in step (iii), Z₁ and Z₂ are the same and selected from chloro or bromo, especially bromo.

Any suitable solvent may be used for this process steps (ii) and (iii) of this process and a person skilled in the art will be able to select suitable reaction conditions.

Compounds in which L is -CH₂-CH₂- can also be formed by:
(i) reacting a compound of formula D1 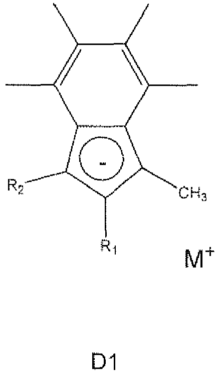 (wherein M is lithium, sodium, or potassium; and R₁ and R₂ are as defined hereinbefore) with BrCN in the presence of a suitable solvent to form a compound of formula E1 shown below 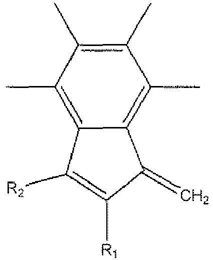 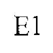 and
(ii) reacting a compound of formula E1 with C₁₀H₈.M in the presence of a suitable solvent to form a compound of formula A

Compounds of formula D1 can be readily synthesized by techniques well known in the art.

Any suitable solvent may be used for step (i) of the above process. A particularly suitable solvent is diethyl ether.

Similarly, any suitable solvent may be used for step (ii) of the above process. A suitable solvent may be, for example, toluene, THF, DMF etc.

For the avoidance of doubt, the C₁₀H₈.M reagent used in step (ii) of the above process is lithium, sodium or potassium naphthalenide. In an embodiment, C₁₀H₈.M is sodium naphthalenide.

The process steps (ii) and (iii) are defined further in the art. For example, reference can be made to WO2011/051705 (see page 11, line 22 to page 14, line 13 and the examples - page 17, line 11 to page 19, line 1).

In addition, a process for the synthesis of a di-sodium ethylene-bis-hexamethylindenyl ligand is described in J. Organomet. Chem., 694, (2009), 1059-1068.

According to a fifth aspect of the present invention there is provided a process for forming a compound of formula III 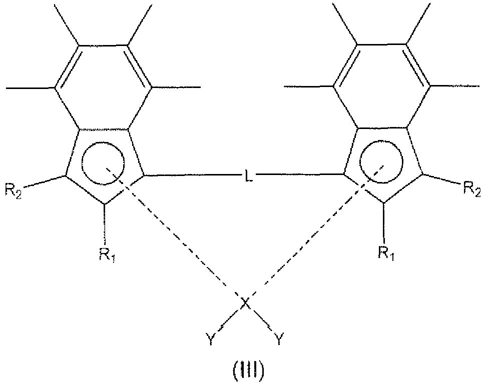
wherein R₁, R₂ and L are as defined herein;
X is zirconium, hafnium or titanium;
Y is selected from halo, hydride, a phosphonated or sulfonated anion, or a (1-6C)alkyl, (1-6C)alkoxy, aryl or aryloxy group which is optionally substituted with halo, nitro, amino, phenyl, (1-6C)alkoxy, or Si[(1-4C)alkyl]₃.
wherein the process comprises:
   (i) forming a compound of formula II by the process defined above;
   (ii) reacting the compound of formula If with a compound of the formula:

      X(Y)₂(Z₃)₂

      wherein X and Y are as defined above and Z₃ is a suitable leaving group (e.g. chloro).

Step (i) of this process is defined above.

Step (ii) of this process typically involves forming a compound for formula IIa: 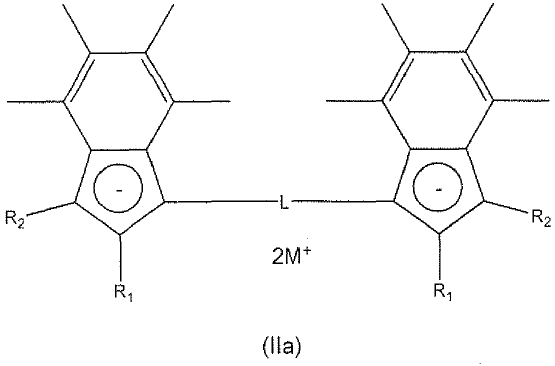
wherein R₁, R₂, M and L are as defined above;
prior to the reaction with X(Y)₂(Z₃)₂.

Suitable techniques for forming compounds of the formula IIa are known in the art (see for example, WO2011/051705). Suitably, the compound of formula IIa is formed by reacting a compound of formula II with a compound MQ as defined hereinbefore (e.g. n-butyllithium).

In step (ii) of this process, Y is a ligand as defined herein and Z₃ is suitably a halide, such as bromo or chloro, especially chloro.

In an embodiment, Y and Z₃ are the same and are suitably a halide, such as bromo or chloro, especially chloro. Once the compound of formula III has been formed, the compound may be reacted to replace the Y groups with another Y group as defined herein other than halide.

Suitably, M is Li in step (ii) of the process defined above and the compound of formula IIa is formed by reacting the compound of formula II with an organolithium compound of the formula MQ.

In an embodiment, the compound X(Y)₂(Z₃)₂ is provided as a solvate. In particular, the compound may be provided as X(Y)₂(Z₃)₂.THFₚ, where p is an integer (e.g. 2).

Any suitable solvent may be used for step (ii) of the process defined above. A suitable solvent may be, for example, diethyl ether, toluene, THF, dichloromethane, chloroform, hexane DMF, benzene etc.

A person of skill in the art will be able to select suitable reaction conditions (e.g. temperature, pressures, reaction times, agitation etc.) for such a synthesis.

### Applications

As previously indicated, the compounds of formula I are extremely important precursors for the formation of ligands of formula II and metallocene catalysts of formula III defined herein. The catalysts of formula III are particularly useful for the polymerisation of polyethylene.

As discussed hereinbefore, these catalyst compounds exhibit superior catalytic performance when compared with current metallocene compounds used in the polymerisation of α-olefins. In particular, when compared with current metallocene compounds used in the polymerisation of α-olefins, the compounds of the invention exhibit significantly increased catalytic activity. Moreover, polymers produced by α-olefin polymerization in the presence of compounds of the invention are typically of a higher molecular weight than polymers prepared using other catalysts, without an attendant increase in polydispersity. Such materials are highly valued by industry.

Thus, the present invention also provides a compound of formula III when prepared by the processes defined herein.

### EXAMPLE

An example of the invention will now be described by reference to the accompanying figures, in which:
Fig. 1 shows the ¹H NMR spectra of hexamethylindanone (Ind^{#}=O);
Fig. 2 shows the molecular structure of hexamethylindanone (Ind^{#}=O);
Fig. 3 shows the ¹H NMR spectra of hexamethylindene (Ind^{#}H); and
Fig. 4 shows the molecular structure of hexamethylindene, (Ind^{#}H).

### Example 1

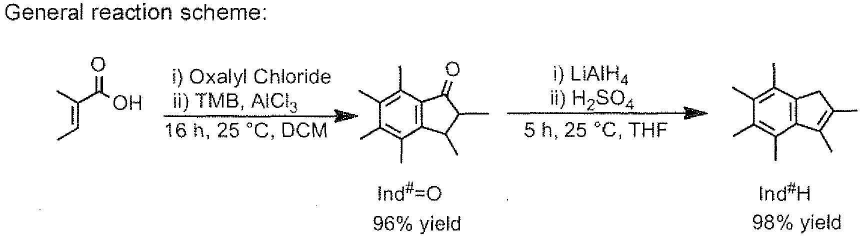

41.8 g tiglic acid [trans-2-methylbutenoic acid] (417 mmol, 1 eq.) was dissolved in 1 L DCM. To this mixture, 52.9 g oxalyl chloride (417 mmol, 1 eq.) was added which was washed in with a 100 mL portion of dichloromethane (DCM). Five drops of N,N-dimethylformamide was pipetted into the vigorously stirred solution causing a large amount of effervescence. The reaction was left stirring under nitrogen for 12 h before cooling to 8 °C, 61.2 g aluminium trichloride (459 mmol, 1.1 eq.) was quickly added under a flow of N₂, causing a small temperature rise and the resulting DCM slurry to become light orange. 50 g 1,2,3,4-tetramethylbenzene (TMB) (372 mmol, 0.90 eq.) was mixed with 100 mL DCM and transferred to a pressure-equalising dropping-funnel. The TMB solution was added to the stirred reaction mixture in a fast-dropwise manner effecting a colour change through dark-orange to red. After stirring for a further four hours at room temperature, a solution of 500 mL conc. HCl and 500 g ice was made-up and was transferred to the reaction mixture to quench it, dropwise at first and then more quickly over 10 minutes. This caused the dark-red slurry to decolourise to a light pink solution. The stirring was stopped and the DCM layer was decanted before extracting the aqueous layer with 500 mL DCM twice more. The combined organic layer was washed with 500 mL deionised water, drying with magnesium sulfate, filtering and reducing *in vacuo* (40 °C, 550 mbar). The product was obtained as a light brown oil in 96 % yield (77.25 g, 357 mmol).

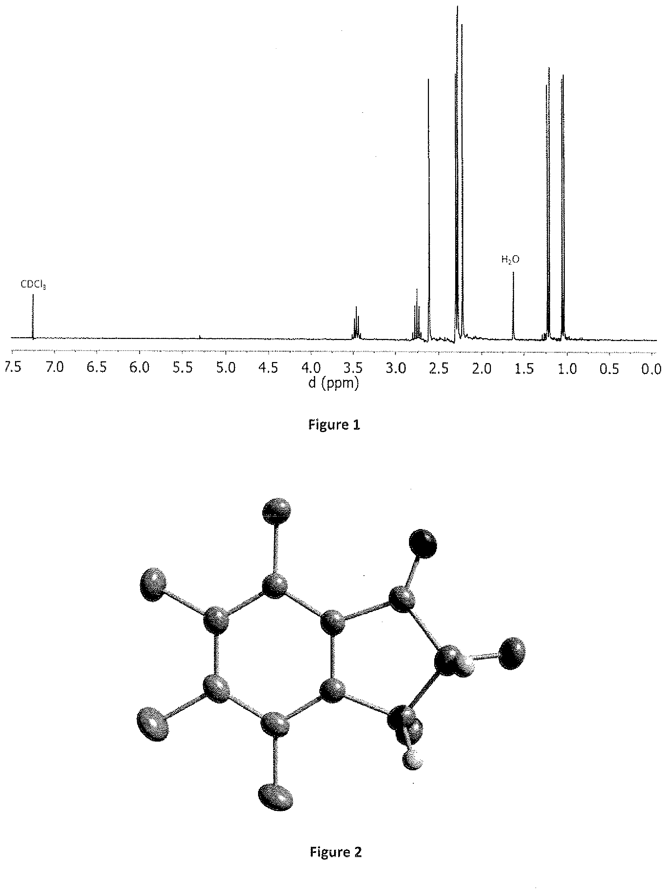

The proton NMR for the Ind^{#}=O is shown in Figure 1 and the molecular structure is shown in Figure 2.

100 mL LiAlH₄ (2.0 M in THF; 200 mmol, 0.5 eq.) was combined with 100 mL dry, degassed THF and the mixture was cooled to 8 °C under N₂. 86.53 g Ind^{#}=O (400 mmol, 1 eq.) was dissolved in 150 mL dry, degassed THF and this was added to the stirred reaction mixture in a fast dropwise manner over the course of 10 minutes causing a small rise in temperature. The reaction was stirred for four hours before careful, portionwise quenching with 14.4 mL H₂O (800 mmol, 2 eq.) over approximately 30 minutes. 113 mL conc. H₂SO₄ (>95 %; 2 mol, 5 eq.) was added, slowly at first, causing the colour to change to a medium-dark grey over 30 minutes. The reaction was quenched with an additional 500 mL H₂O and extracted with DCM (3x 500 mL). The combined organic layer was washed with a further 500 mL H₂O, dried over magnesium sulphate and reduced *in vacuo* (40 °C, 250 mbar), affording the product as a dark brown oil in 98 % yield (78.51 g, 392 mmol).

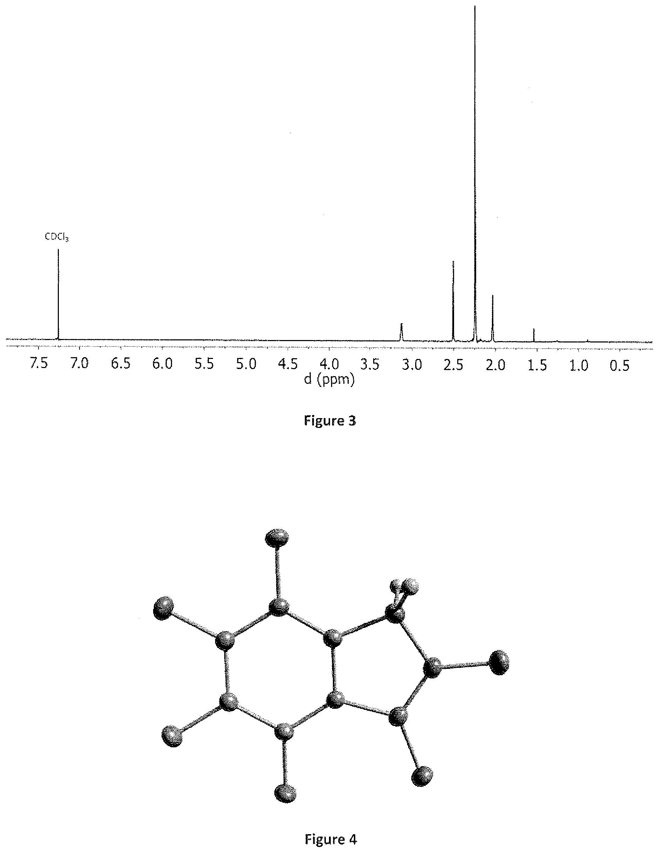

The proton NMR for the Ind^{#}H is shown in Figure 3 and the molecular structure is shown in Figure 4.

### EXAMPLE 2

### This synthesis is based on R₁ = CH₃ and R₂ = C₂H₅

### Synthesis of 3-ethyl-tigloyl chloride

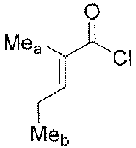

### (E)-2-methylpent-2-enoyl chloride

One equivalent of oxalyl chloride (26.45 g, 208 mmol) was added to a 2 L reaction vessel containing one equivalent of (E)-2-methylpent-2-enoic acid (23.85 g, 208 mmol) in DCM (500 mL) under a flow of N₂. While stirring, five drops of dry DMF were pipetted into the mixture creating effervescence. The reaction was left in a state a reflux for 2 hours. An aliquot was taken after 90 minutes showing that the reaction had gone to completion.
**¹H NMR (CDCl₃):** δ 1.08 (t, 3H, *J* = 7.6 Hz, **Me**_{b}), δ 1.88 (q, 3H, *J* = 1.0 Hz, **Me**ₐ), δ 2.26 (quinq, 2H, *J* = 7.5, 0,9 Hz, **CH₂),** δ 6.87 (tq, 1H, *J* = 7.4 Hz, 1.3 Hz, vinylic-**H**)
**¹³C{¹H} NMR (CDCl₃):** δ 12.57 **(Me**_{b}**),** δ 13.23 (**Me**ₐ), δ 22.93 (**CH₂**), δ 154.23 (Vinylic-**H**)

### Synthesis of (Ind^{#,3-Ethyl})=o

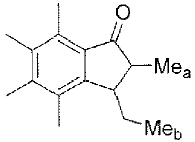

### 3-ethyl-2,4,5,6,7 -pentamethyl-2,3-dihydro-1H-inden-1-one

The reactor was cooled to 8°C and allowed to equilibrate. 1.1 equivalents of aluminium trichloride (30.6 g, 230 mmol) was added, under a flow of N₂, to the reactor. The mixture changed from a pale yellow to a deep orange almost instantly. 0.9 equivalents of tetramethylbenzene (25.0 g, 176 mmol) was diluted in 100 mL DCM and transferred to a pressure equalising funnel. This mixture was added to the reaction vessel dropwise over 15 minutes where a colour change from deep orange to blood red was observed. The solution was then left to stir for two hours, after which a solution of 100 mL conc. HCl and 100 g ice was made up and used to quench the reaction. The reaction mixture changed colour from blood red to a light orange solution during this workup. The product was extracted with DCM (3 x 100 mL) and the combined organic layer washed with deionised water (3 x 100 mL) before being dried using anhydrous MgSO₄. This was filtered and the DCM solvent removed *in vacuo* to afford a beige solid in 100% yield (41.8 g, 214 mmol).
**¹H NMR (CDCl₃):** δ 0.56 (t, 3H, *J* = 7.5 Hz, **Me**_{b}), δ 1.28 (d, 3H, *J* = 7.2 Hz, **Me**ₐ),
δ 1.70 (m, 2H, **CH₂),** δ 2.23 (s, 3H, Ar-**Me**), δ 2.28 (s, 3H, Ar-**Me**), δ 2.28 (s, 3H, Ar-**Me**), δ 2.62 (s, 3H, Ar-**Me**), δ 2.74 (quin, 1H, *J* = 7.3 Hz, C**H**), δ 2.74 (quin, 1H, *J* = 7.3 Hz, C**H**), δ 3.45 (ddd, 1H, *J* = 7,4, 6.1, 3.6 Hz, C**H**)
**MS (ESI):** found 231.17446; calculated 231.17434.

### Synthesis of (Ind^{#,3-ethyl})H

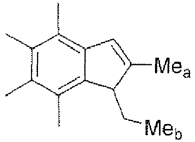

### 1-ethyl-2,4,5,6,7-pentamethyl-1H-indene

One equivalent of 3-ethyl-2,4,5,6,7-pentamethyl-2,3-dihydro-1H-inden-1-one (22.4 g, 97 mmol) was added to 50 mL of dry, degassed THF in a schlenk tube. 0.5 equivalents of LiAlH₄ (24.2 mL, 48 mmol) were added dropwise over 30 minutes and the resultant mixture left to stir for 2 hours under nitrogen. This caused the reaction mixture to turn light orange. The schlenk was cooled to 0°C and the reaction quenched by adding one equivalent of deionised water (1.74 mL, 97 mmol) dropwise. Upon addition of five equivalents of conc. H₂SO₄ (26.1 mL, 483 mmol) dropwise over 20 minutes the reaction mixture turned dark brown. The resultant solution was transferred to a 2 L reaction vessel and stirred for 30 minutes under a flow of N₂. The reaction was then quenched with 250 mL deionised water and extracted with DCM (3 x 500 mL). The combined organic layer was washed with deionised water (3 x 100 mL), dried over anhydrous MgSO₄, filtered, and the solvent removed *in vacuo* to give a light brown solid (16.8g, 78 mmol) in 80.6% yield.

**¹H NMR (CDCl₃):** δ 0.46 (t, 3H, *J* = 7.4 Hz, **Me**_{b}), δ 1.97 (dqd, 1H, *J* = 13.9, 7.4, 3.7 Hz, C**H**), δ 2.09 (d, 1H, *J* = 0.8 Hz, **Me**ₐ) δ 2.14 (m, 1H, C**H**), δ 2.29 (s, 3H, Ar-**Me**), δ 2.29 (s, 3H, Ar-**Me**), δ 2.36 (s, 3H, Ar-**Me**), δ 2.37 (s, 3H, Ar-**Me**), δ 3.36 (t, 1 H, *J* = 4.5 Hz, CH), δ 6.62 (quin, 1H, *J* = 1.5 Hz)
**MS (ESI):** found 215.17966; expected 215.17943.

### Synthesis of Ind^{#,3-ethyl}Li

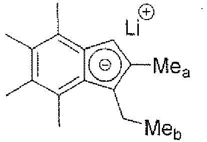

### 1-ethyl-2,4,5,6,7-pentamethyl Indenyllithium

One equivalent of 1-ethyl-2,4,5,6,7-pentamethyl-1H-indene (16.8 g, 78 mmol) was dissolved in 50 mL DCM and transferred to a schlenk tube. The solvent was removed *in vacuo* before 100 mL pentane was added. 1.1 equivalents of *n*-butyllithium (34.5 mL, 86 mmol) was added dropwise, at 0°C, to the dark brown mixture while stirring. The solution was allowed to warm up to room temperature and left stirring for 16 hours. The resulting yellow suspension was filtered on an air sensitive frit. The powder was washed with pentane (2 x 50 mL) and then dried under vacuum to form a white solid in 52.4% yield (9.05 g, 41 mmol)
**¹H NMR (C₅D₅N):** δ 1.47 (t, 3H, *J* = 7.3 Hz, **Me**_{b}), δ 2.45 (s, 3H, Ar-**Me**), δ 2.46 (s, 3H, Ar-**Me**), δ 2.65 (s, 3H, Ar-**Me**), δ 2.66 (s, 3H, Ar-**Me**), δ 2.89 (s, 3H, **Me**ₐ), δ 3.31 (q, 2H, *J* = 7.3 Hz, **CH₂**), δ 6.37 (s, 1H, Ar-**H**)
**⁷Li NMR (C₅D₅N):** δ 1.06 (s)

### Synthesis of (Ind^{*,3-ethyl})₂ZrCl₂

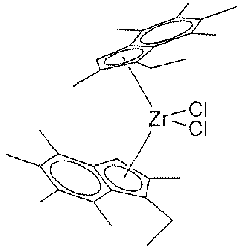

Two equivalents of Ind^{#,3-ethyl}Li (3 g, 13.6 mmol) were added to one equivalent of ZrCl₄ (1.59 g, 6.84 mmol) in a schlenk tube inside a glovebox, and were stirred in 100 mL benzene under nitrogen for 16 hours at room temperature. The mixture was allowed to settle, and the mixture filtered. The solution was dried to afford an orange solid in 27.0% yield (1.09 g, 1.85 mmol). This solid comprised an equal mixture of both *rac-* and *meso-*isomeric forms which proved inseparable by fractional crystallisation from hexane, pentane, Et₂O and benzene. A further recrystallization from toluene yielded a light yellow precipitate of *rac*-(Ind^{*,3-ethyl})₂ZrCl₂ which was isolated.
**¹H NMR (C₆D₆):** δ 1.00 (t, 6H, *J* = 7.6 Hz, **Me**), δ 1.58 (s, 6H, Ar-**Me**), δ 2.09 (s, 6H, Ar-**Me**), δ 2.16 (s, 6H, Ar-**Me**), δ 2.38 (s, 6H, Ar-**Me**), δ 2.60 (s, 6H, Ar-**Me**), δ 2.81 (dq, 2H, *J* = 15.3, 7.7 Hz, **CH₂),** δ 3.31 (dq, 2H, *J* = 15.1, 7.5 Hz, **CH₂**), δ 6.09 (s, 2H, Ar-**H**)

The features disclosed in the foregoing description, in the claims and the accompanying drawings may, both separately and in any combination, be material for realizing the invention in diverse forms thereof.

## Claims

1. A process for the preparation of a compound of the formula I shown below: 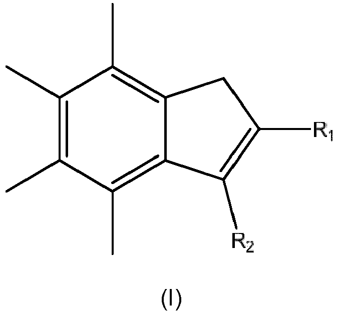 wherein:
R₁ and R₂ are each (1-10C)alkyl;
and wherein the process comprises the steps of:
(i) reacting a compound of formula A: 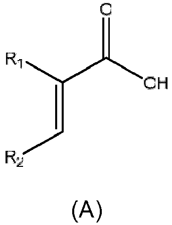
wherein R₁ and R₂ are each as defined above;
with a chlorinating or brominating agent to form a compound of formula B: 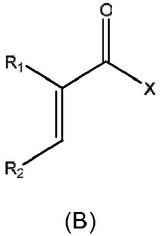 wherein R₁ and R₂ are each as defined above and X is chloro or bromo;
and adding 1,2,3,4-tetramethylbenzene and a Lewis acid catalyst to the reaction mixture to react with compound B to form a compound of formula C: 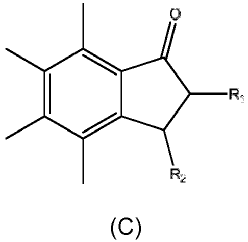 wherein R₁ and R₂ are each as defined above;
(ii) reacting the compound of formula C with a solution of a hydride transfer reagent followed by adding a dehydrating agent to the reaction mixture to form a compound of Formula I.

2. The process according to claim 1, wherein
(a) R₁ and R₂ are each independently selected from (1-3C)alkyl; or
(b) R₁ and R₂ are both methyl.

3. The process according to claim 1 or 2, wherein in step (i) a chlorinating agent is used and the chlorinating agent is selected from the group consisting of oxalyl chloride, PCl₃ and PCl₅.

4. The process according to any one of claims 1, 2 and 3, wherein the chlorinating agent is oxalyl chloride.

5. The process according to any one of the preceding claims, wherein
(a) the Lewis acid catalyst is selected from AlCl₃, AlBr₃ and BCl₃; or
(b) the Lewis acid catalyst is AlCl₃.

6. The process according to any one of the preceding claims, wherein the reaction between the compound of formula B and 1,2,3,4-tetramethylbenzene is quenched by the addition of an acid.

7. The process according to any one of the preceding claims, wherein
(a) in step (ii) the solution of a hydride transfer reagent is selected from a solution of LiAlH₄ and NaBH₄; or
(b) in step (ii) the solution of a hydride transfer reagent is a solution of LiAlH₄.

8. The process according to any one of the preceding claims, wherein in step (ii) the dehydrating agent is an acid selected from the group consisting of sulphuric, hydrochloric or phosphoric acid.

9. A process for forming a compound of formula II 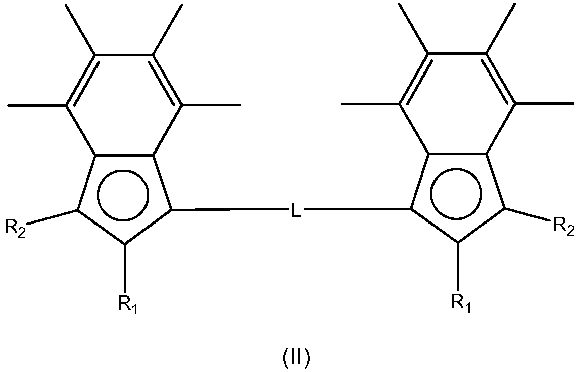 wherein
R₁, R₂ are as defined in claim 1; and
L is a bridging group of the formula -[C(R^{x}R^{y})]ₙ- wherein n is 1, 2 or 3 and R^{x} and R^{y} are each independently hydrogen, (1-6C)alkyl, (2-6C)alkenyl, (2-6C)alkynyl or (1-6C)alkoxy; or a group -SiRₐR_{b} wherein Rₐ and R_{b} are each independently selected from (1-6C)alkyl, (2-6C)alkenyl, (2-6C)alkynyl, (1-6C)alkoxy or phenyl;
wherein the process comprises:
(i) forming a compound of formula I by a process as defined in claims 1 to 8;
(ii) reacting the compound of formula I with an organolithium, organosodium or organopotassium compound of the formula:
MQ
wherein M is lithium, sodium, or potassium and Q is an (1-6C)alkyl or aryl group; to form a compound of formula D shown below: 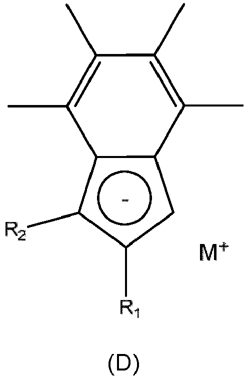
(iii) reacting two equivalents of a compound of formula D shown above with one equivalent of a compound having formula E shown below:
Z₁-L-Z₂ (E)
wherein L is as defined above and Z₁ and Z₂ are leaving groups to form a compound of formula II.

10. The process according to claim 9, wherein L is -CH₂-CH₂-, -CH₂-CH₂-CH₂- or a group -SiRₐR_{b} wherein Rₐ and R_{b} are each independently selected from methyl, propyl and allyl.

11. The process according to claim 9 or claim 10, wherein in step (ii) the compound of formula I is reacted with an organolithium compound (i.e. M is lithium).

12. The process according to claim 11, wherein the organolithium compound is n-butyllithium.

13. The process according to any one of claims 9 to 12, wherein Z₁ and Z₂ are the same and selected from chloro or bromo.

14. A process for forming a compound of formula III 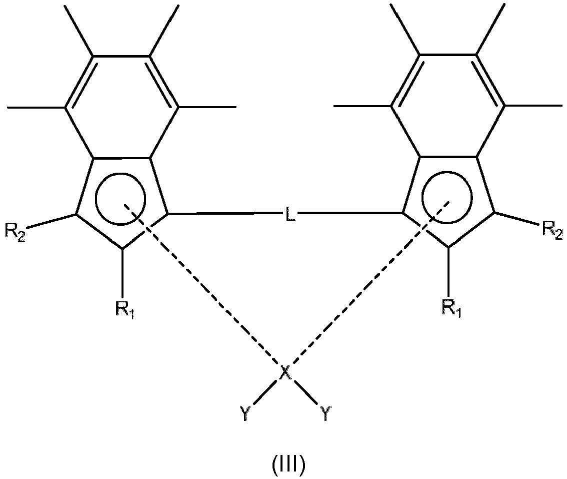 wherein
R₁, R₂ are as defined in claims 1 or 2;
L is as defined in claims 9 or 10;
X is zirconium, hafnium or titanium;
Y is selected from halo, hydride, a phosphonated or sulfonated anion, or a (1-6C)alkyl, (1-6C)alkoxy, aryl or aryloxy group which is optionally substituted with halo, nitro, amino, phenyl, (1-6C)alkoxy, or Si[(1-4C)alkyl]₃,
wherein the process comprises:
(i) forming a compound of formula II by the process defined in claims 9 to 13 above;
(ii) reacting the compound of formula II, or a salt thereof, with a compound of the formula:
X(Y)₂(Z₃)₂
wherein X and Y are as defined above and Z₃ is a leaving group.

15. The process according to claim 14, wherein Z₃ is a halide,
and optionally wherein X is zirconium or hafnium.

## Patentansprüche

1. Verfahren zur Herstellung einer nachstehend dargestellten Verbindung der Formel I: 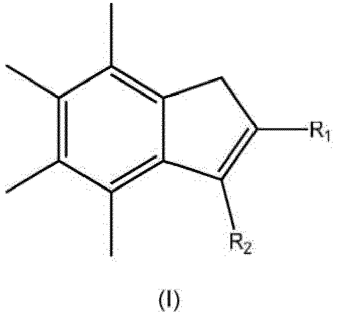 wobei:
R₁ und R₂ jeweils (1-10C)Alkyl sind;
und wobei das Verfahren die folgenden Schritte umfasst:
(i) Reagierenlassen einer Verbindung der Formel A: 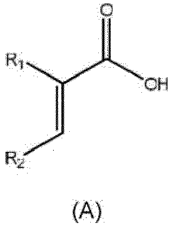
wobei R₁ und R₂ jeweils wie vorstehend definiert sind;
mit einem Chlorierungs- oder Bromierungsmittel, um eine Verbindung der Formel B zu bilden: 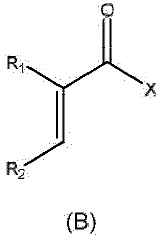 wobei R₁ und R₂ jeweils wie vorstehend definiert sind und X Chlor oder Brom ist;
und Addieren von 1,2,3,4-Tetramethylbenzol und eines Lewissäure-Katalysators zum Reaktionsgemisch zur Reaktion mit Verbindung B, um eine Verbindung der Formel C zu bilden: 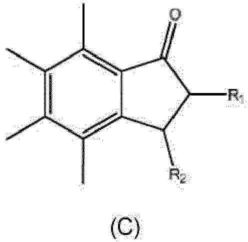 wobei R₁ und R₂ jeweils wie vorstehend definiert sind;
(ii) Reagierenlassen der Verbindung der Formel C mit einer Lösung eines Hydridtransferreagens gefolgt vom Addieren eines Dehydratisierungsmittels zum Reaktionsgemisch, um eine Verbindung der Formel I zu bilden.

2. Verfahren nach Anspruch 1, wobei
(a) R₁ und R₂ jeweils unabhängig ausgewählt sind aus (1-3C)Alkyl; oder
(b) R₁ und R₂ beide Methyl sind.

3. Verfahren nach Anspruch 1 oder 2, wobei in Schritt (i) ein Chlorierungsmittel verwendet wird und das Chlorierungsmittel ausgewählt ist aus der Gruppe bestehend aus Oxalylchlorid, PCl₃ und PCl₅.

4. Verfahren nach einem der Ansprüche 1, 2 und 3, wobei das Chlorierungsmittel Oxalylchlorid ist.

5. Verfahren nach einem der vorhergehenden Ansprüche, wobei
(a) der Lewissäure-Katalysator ausgewählt ist aus AlCl₃, AlBr₃ und BCl₃, oder
(b) der Lewissäure-Katalysator AlCl₃ ist.

6. Verfahren nach einem der vorhergehenden Ansprüche, wobei die Reaktion zwischen der Verbindung der Formel B und 1,2,3,4-Tetramethylbenzol durch die Addition einer Säure gequencht wird.

7. Verfahren nach einem der vorhergehenden Ansprüche, wobei
(a) in Schritt (ii) die Lösung eines Hydridtransferreagens ausgewählt ist aus einer Lösung von LiAlH₄ und NaBH₄; oder
(b) in Schritt (ii) die Lösung eines Hydridtransferreagens eine Lösung von LiAlH₄ ist.

8. Verfahren nach einem der vorhergehenden Ansprüche, wobei in Schritt (ii) das Dehydratisierungsmittel eine Säure ist, die ausgewählt ist aus der Gruppe bestehend aus Schwefel-, Salz- oder Phosphorsäure.

9. Verfahren zum Bilden einer Verbindung der Formel II 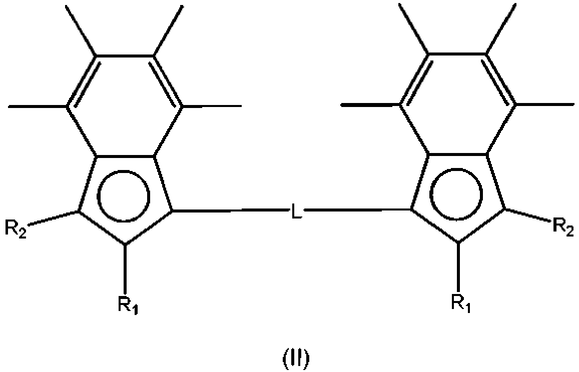 wobei
R₁, R₂ wie in Anspruch 1 definiert sind; und
L eine Überbrückungsgruppe der Formel -[C(R^{x}R^{y})]ₙ- ist, wobei n 1, 2 oder 3 ist und R^{x} und R^{y} jeweils unabhängig Wasserstoff, (1-6C)Alkyl, (2-6C)Alkenyl, (2-6C)Alkynyl oder (1-6C)Alkoxy sind; oder eine Gruppe -SiRₐRb, wobei Rₐ und R_{b} jeweils unabhängig ausgewählt sind aus (1-6C)Alkyl, (2-6C)Alkenyl, (2-6C)Alkynyl, (1-6C)Alkoxy oder Phenyl;
wobei das Verfahren umfasst:
(i) Bilden einer Verbindung der Formel I durch ein Verfahren wie in Ansprüchen 1 bis 8 definiert;
(ii) Reagierenlassen der Verbindung der Formel I mit einer Organolithium-, Organonatrium- oder Organokalium-Verbindung der Formel:
MQ
wobei M Lithium, Natrium oder Kalium ist und Q eine (1-6C)Alkyl- oder Arylgruppe ist; um eine nachstehend dargestellte Verbindung der Formel D zu bilden: 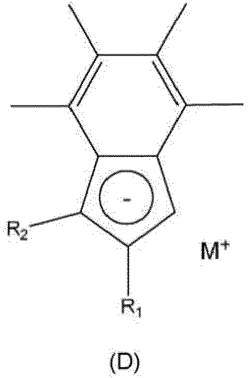
(iii) Reagierenlassen zweier Äquivalente einer vorstehend dargestellten Verbindung der Formel D mit einem Äquivalent einer nachstehend dargestellten Verbindung der Formel E:
Z₁-L-Z₂ (E)
wobei L wie vorstehend definiert ist und Z₁ und Z₂ Abgangsgruppen zum Bilden einer Verbindung der Formel II sind.

10. Verfahren nach Anspruch 9, wobei L -CH₂-CH₂-, -CH₂-CH₂-CH₂- oder eine Gruppe -SiRₐR_{b} ist, wobei Rₐ und R_{b} jeweils unabhängig ausgewählt sind aus Methyl, Propyl und Allyl.

11. Verfahren nach Anspruch 9 oder Anspruch 10, wobei in Schritt (ii) die Verbindung der Formel I mit einer Organolithium-Verbindung in Reaktion gebracht wird (d. h. M ist Lithium).

12. Verfahren nach Anspruch 11, wobei die Organolithium-Verbindung n-Butyllithium ist.

13. Verfahren nach einem der Ansprüche 9 bis 12, wobei Z₁ und Z₂ identisch und aus Chlor oder Brom ausgewählt sind.

14. Verfahren zum Bilden einer Verbindung der Formel III 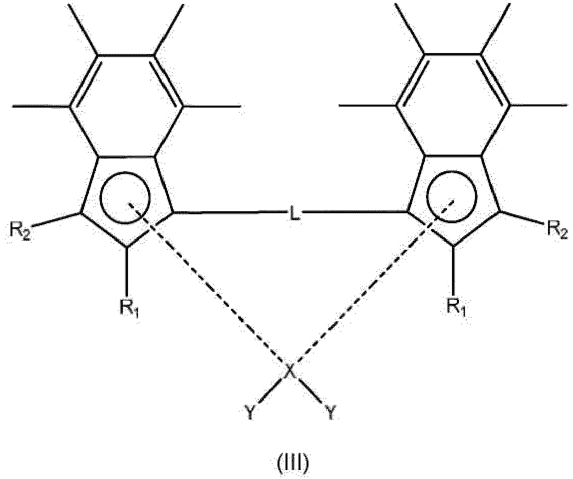 wobei
R₁, R₂ wie in Anspruch 1 oder 2 definiert sind;
L wie in Anspruch 9 oder 10 definiert ist;
X Zirkonium, Hafnium oder Titan ist;
Y ausgewählt ist aus Halo, Hydrid, einem phosphonierten oder sulfonierten Anion oder einer (1-6C)Alkyl-, (1-6C)Alkoxy-, Aryl- oder Aryloxygruppe, die wahlweise mit Halo, Nitro, Amino, Phenyl, (1-6C)Alkoxy oder Si[(1-4C)Alkyl]₃ substituiert ist,
wobei das Verfahren umfasst:
(i) Bilden einer Verbindung der Formel II durch das in vorstehenden Ansprüchen 9 bis 13 definierte Verfahren;
(ii) Reagierenlassen der Verbindung der Formel II, oder eines Salzes davon, mit einer Verbindung der Formel:
X(Y)₂(Z₃)₂
wobei X und Y vorstehend definiert sind und Z₃ eine Abgangsgruppe ist.

15. Verfahren nach Anspruch 14, wobei Z₃ ein Halid ist,
und wahlweise, wobei X Zirkonium oder Hafnium ist.

## Revendications

1. Procédé de préparation d'un composé de formule I représenté ci-dessous : 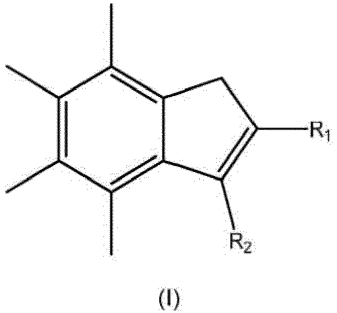 dans laquelle :
R₁ et R₂ sont chacun alkyle en (C1 à 10) ;
et dans lequel le procédé comprend les étapes de :
(i) mise en réaction d'un composé de formule A : 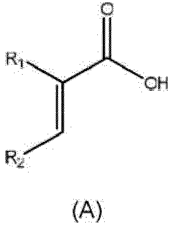 dans laquelle R₁ et R₂ sont chacun tels que définis ci-dessus ;
avec un agent de chloration ou de bromation pour former un composé de formule B : 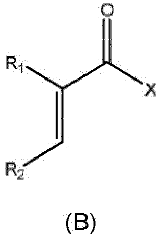 dans laquelle R₁ et R₂ sont chacun tels que définis ci-dessus et X est chloro ou bromo ;
et l'ajout de 1,2,3,4-tétraméthylbenzène et d'un catalyseur d'acide de Lewis au mélange réactionnel pour réagir avec le composé B pour former un composé de formule C : 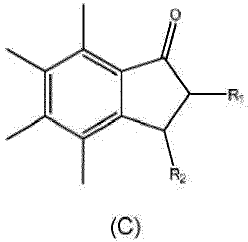 dans laquelle R₁ et R₂ sont chacun tels que définis ci-dessus ;
(ii) mise en réaction du composé de formule C avec une solution d'un réactif de transfert d'hydrure suivie par l'ajout d'un agent de déshydratation au mélange réactionnel pour former un composé de formule I.

2. Procédé selon la revendication 1, dans lequel
(a) R₁ et R₂ sont chacun indépendamment sélectionnés parmi l'alkyle en (C1 à 3) ; ou
(b) R₁ et R₂ sont tous les deux méthyle.

3. Procédé selon la revendication 1 ou 2, dans lequel dans l'étape (i) un agent de chloration est utilisé et l'agent de chloration est sélectionné dans le groupe constitué par le chlorure d'oxalyle, PCl₃ et PCl₅.

4. Procédé selon l'une quelconque des revendications 1, 2 et 3, dans lequel l'agent de chloration est le chlorure d'oxalyle.

5. Procédé selon l'une quelconque des revendications précédentes, dans lequel
(a) le catalyseur d'acide de Lewis est sélectionné parmi AlCl₃, AlBr₃ et BCl₃ ; ou
(b) le catalyseur d'acide de Lewis est AlCl₃.

6. Procédé selon l'une quelconque des revendications précédentes, dans lequel la réaction entre le composé de formule B et le 1,2,3,4-tétraméthylbenzène est éteinte par l'ajout d'un acide.

7. Procédé selon l'une quelconque des revendications précédentes, dans lequel
(a) dans l'étape (ii) la solution d'un réactif de transfert d'hydrure est sélectionnée par une solution de LiAlH₄ et de NaBH₄ ; ou
(b) dans l'étape (ii) la solution d'un réactif de transfert d'hydrure est une solution de LiAlH₄.

8. Procédé selon l'une quelconque des revendications précédentes, dans lequel dans l'étape (ii) l'agent de déshydratation est un acide sélectionné dans le groupe constitué par l'acide sulfurique, chlorhydrique ou phosphorique.

9. Procédé de formation d'un composé de formule II 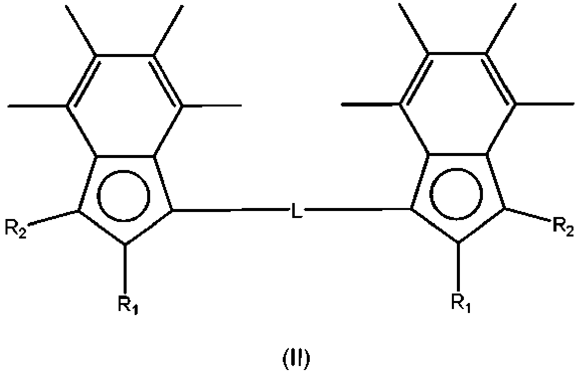 dans laquelle
R₁, R₂ sont tels que définis dans la revendication 1 ; et
L est un groupe de pontage de formule -[C(R^{x}R^{y})]ₙ- dans laquelle n est 1, 2 ou 3 et R^{x} et R^{y} sont chacun indépendamment hydrogène, alkyle en (C1 à 6), alcényle en (C2 à 6), alcynyle en (C2 à 6) ou alcoxy en (C1 à 6) ; ou un groupe -SiRₐR_{b} dans lequel Rₐ et R_{b} sont chacun indépendamment sélectionnés parmi l'alkyle en (C1 à 6), l'alcényle en (C2 à 6), l'alcynyle en (C2 à 6), l'alcoxy en (C1 à 6) ou le phényle ;
dans lequel le procédé comprend :
(i) la formation d'un composé de formule I par un procédé tel que défini dans les revendications 1 à 8 ;
(ii) la mise en réaction du composé de formule I avec un composé organolithium, organosodium ou organopotassium de formule :
MQ
dans laquelle M est du lithium, du sodium, ou du potassium et Q est un groupe alkyle en (C1 à 6) ou un groupe aryle ;
pour former un composé de formule D représenté ci-dessous : 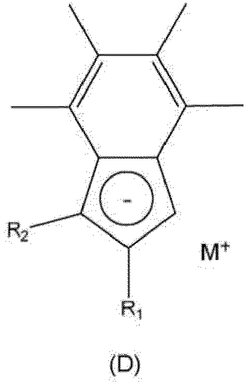
(iii) la mise en réaction de deux équivalents d'un composé de formule D représenté ci-dessus avec un équivalent d'un composé de formule E représenté ci-dessous :
Z₁-L-Z₂ (E)
dans laquelle L est tel que défini ci-dessus et Z₁ et Z₂ sont des groupes labiles pour former un composé de formule II.

10. Procédé selon la revendication 9, dans lequel L est -CH₂-CH₂-, -CH₂-CH₂-CH₂- ou un groupe -SiRₐR_{b} dans lequel Rₐ et R_{b} sont chacun indépendamment sélectionnés parmi le méthyle, le propyle et l'allyle.

11. Procédé selon la revendication 9 ou la revendication 10, dans lequel dans l'étape (ii) le composé de formule I est mis à réagir avec un composé organolithium (à savoir, M est du lithium).

12. Procédé selon la revendication 11, dans lequel le composé organolithium est le n-butyllithium.

13. Procédé selon l'une quelconque des revendications 9 à 12, dans lequel Z₁ et Z₂ sont les mêmes et sélectionnés parmi chloro ou bromo.

14. Procédé de formation d'un composé de formule III 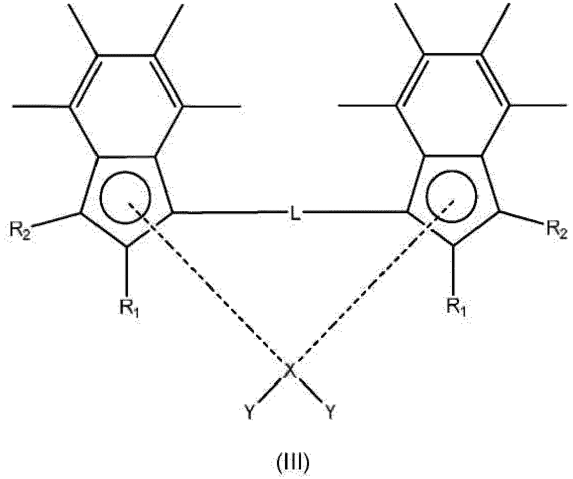 dans laquelle
R₁, R₂ sont tels que définis dans les revendications 1 ou 2 ;
L est tel que défini dans les revendications 9 ou 10 ;
X est le zirconium, l'hafnium ou le titane ;
Y est sélectionné parmi l'halo, l'hydrure, un anion phosphoné ou sulfoné, ou un groupe alkyle en (C1 à 6), alcoxy en (C1 à 6), aryle ou aryloxy qui est facultativement substitué avec halo, nitro, amino, phényle, alcoxy en (C1 à 6), ou Si[alkyle en (C1 à 4)]₃,
dans lequel le procédé comprend :
(i) la formation d'un composé de formule II par le procédé défini dans les revendications 9 à 13 ci-dessus ;
(ii) la réaction du composé de formule II, ou d'un sel de celui-ci, avec un composé de formule :
X(Y)₂(Z₃)₂
dans laquelle X et Y sont tels que définis ci-dessus et Z₃ est un groupe labile.

15. Procédé selon la revendication 14, dans lequel Z₃ est un halogénure, et facultativement dans lequel X est le zirconium ou l'hafnium.
